# EUROPEAN PATENT APPLICATION

(11) **EP 1 902 728 A1**
(43) Date of publication of application: **26.03.2008**
(21) Application number: 06076745.6
(22) Date of filing: 19.09.2006
(51) Int. Cl.: A61K 41/00, A61L 27/44, A61L 27/34

(54) **Medical device made of a bioresorbable composition**

(71) Applicant: Stichting Katholieke Universiteit, meer in het bijzonder het Universitair Medisch Centrum St Radboud, 6500 HB Nijmegen (NL)
(72) Inventor: Verdonschot, Nicolaas, Jacobus, Joseph, 6524 WX Nijmegen (NL); van Hest, Jan, Cornelis, Maria, 6522 DL Nijmegen (NL); Buma, Pieter, 6500 HB Nijmegen (NL); Ayres, Lee, 6521 DA Nijmegen (NL); Vriezema, Dennis, M., 6500 HB Nijmegen (NL)
(74) Representative: van Loon, C.J.J.

(57) **Abstract**

The invention provides a bioresorbable composition comprising a mixture of at least one polymer and capsules, which capsules comprise an inner part which is encapsulated by a shell which shell is at least partly decomposable by application of an external stimulation source. The invention also relates to a medical device comprising said composition.

## Description

The present invention relates to a bioresorbable composition and a medical device comprising said composition.

In the past most of the devices used in facial reconstruction, fracture treatment and bone defect reconstruction were made of metal and required sometimes a second operation to remove such devices once they had accomplished their objectives.

Fracture healing is a complex process modulated by biological and mechanical factors. Metal plates, screws, pins, and rods are often used to fixate the fracture but are usually quite rigid and reduce the load that is being applied to the bony segments, which is necessary for the repair process. Moreover, often these devices need to be removed at a later stage incurring extra costs (hospitalization, absence from work, etc.) and considerable discomfort to the patients (pain, dangers of anaesthesia, post-operative rehabilitation program).

Particularly for the elderly patient, it is sometimes impossible to remove the device, as the general condition of the patient does not allow this surgical intervention (leading to peri-operative complications or post-operative cognitive dysfunctioning). To overcome these problems, fracture fixation devises made of degradable materials have been developed. These degradable devices are resorbed within the body without the requirement of an additional operation. However, the problem with these devices is that their resorption rate cannot be tuned to the patients requirements, which is important because fracture healing is a complex process, which requires an unpredictable amount of time. For example in diabetic patients a fracture may heal much slower than in a patient with a healthy status.

The objective of the present invention is to provide a bioresorbable composition for use in a medical device that can be implanted and which becomes resorbable at any desired moment of time, thereby allowing each patient individually to be treated according to his/her specific needs.

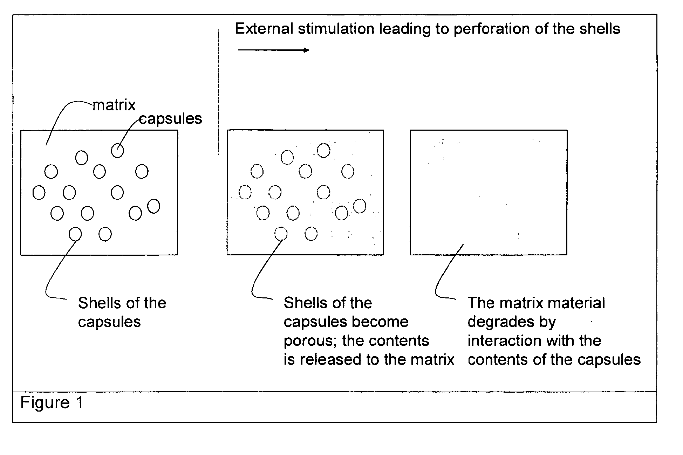

The objective of the present invention can be realised when use is made of a bioresorbable composition that comprises a mixture of a polymer and particular small capsules (Figure 1).

Accordingly, the present invention provides a bioresorbable composition comprising a mixture of at least one polymer and small capsules, which capsules comprise an inner part which is encapsulated by a shell which shell is at least partly decomposable by application of an external stimulation source.

In accordance with the present invention a medical device is removed by the body itself without needing an additional operation. It improves the quality of life of individuals to a considerable degree and, more generally, it will reduce the costs for health care.

Further, the present invention can also be used to release functional agents (like antibiotics, enzymes or proteins) on demand at any point in the post-operative time.

Suitably, the mixture to be used in the present invention has the form of a polymer matrix in which the capsules are embedded or which matrix is surrounded by a coating containing such capsules. The component which contains the capsules, either the matrix or the coating, should preferably be not soluble in body fluid, until said capsules are disrupted.

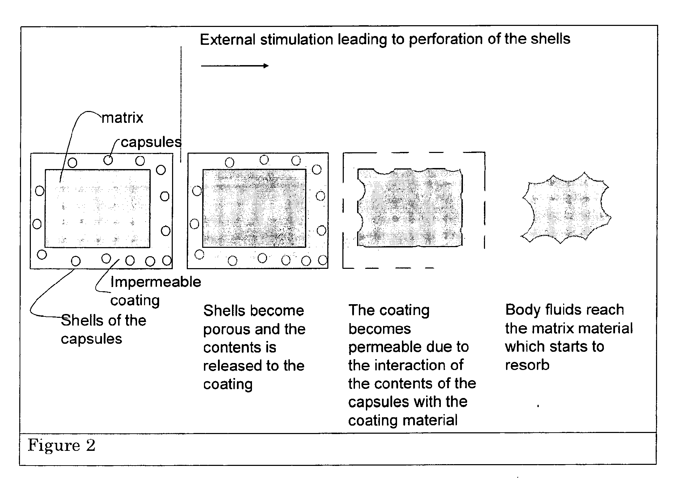

In the composition that the matrix is surrounded by a coating that isolates the matrix from the surrounding biological environment, the resorption characteristics of the device and those of the matrix are decoupled (Figure 2). Hence, the device may sustain long periods in the body even when fastly resorbing matrix materials are used. Therefore the invention allows the use of a broader scale of resorbable materials to be used, and opens the possibility to use stronger, but fastly degradable materials. As such, the present invention opens a whole new perspective to design, develop, optimize and manufacture new devices for the health care industry, surgeons and their patients.

This matrix or coating can consist of any bio-inert or biodegradable polymer, preferably selected from the group consisting of polyesters, polyamides, polyethers, polyurethanes, polyanhydrides and polyphosphazines, polyesters, polylactides and polycarbonates, poly saccharides, poly(ketals), poly(acetals) and polyacrylates.

Suitable examples of polyesters are poly(□-hydroxy acid)s and polylactones, such as poly (lactic acid), poly (glycolic acid), poly(□-propionolactone), poly (hydroxybutyric acid), poly (valeric acid) or poly(valerolactone), polyhexanoic acid, poly (□-caprolactone), and higher homologues such as poly (□-pentadecalactone) The polyester to be used in the matrix or coating is preferably poly (□-caprolactone) or poly (lactic acid).

Suitable examples of poly(amides) are (protected) □-amino acids, such as poly(leucine), poly(valine), poly(glutamic acid), poly benzyl glutamate and proteins such as elastin, collagen and silk. The pol.y(amide) to be used in the matrix or coating is preferably poly(glutamic acid) or poly benzyl glutamate

Suitable examples of poly(anhydrides) are sebacic anhydride, p-(carboxy phenoxy) propane and p-(carboxy phenoxy) hexane.

Suitable examples of poly(ortho esters) are diketene acetals such as 3,9-diethylene 2,4,8,10-tetraoxaspiro[5.5] undecane.

Suitable examples of poly(ethers) are ethylene oxide, propylene oxide, and butylene oxide.

Suitable examples of poly(urethanes) are m-xylylene diisocyanate or hexane diisocyanate and propane diol, ethylene glycol, or poly(ethylene glycol).

Suitable examples of poly(phosphazenes) are amino acid modified dichloro poly(phosphazenes).

Suitable examples of polysaccharides are chitosan, alginate, and cellulose.

Suitable examples of polyacrylates, polymethacrylates and polyacrylamides are acrylic acid, ethyl acrylate, methacrylic acid, N-isopropyl acrylamide, and 2-hydroxyethylmethacrylate.

A suitable example of poly(ketals) is 1,4-phenyleneacetone dimethylene ketal.

Suitable examples of poly(acetals) are oxymethylene and triethylene glycol divinyl ether.

The shells of the capsules can be made out of combinations of any bio inert or biodegradable polymer. To form the shells it is preferred to have a di or tri block copolymer with one or two part(s) consisting of a hydrophilic polymer and the other part(s) consisting of a hydrophobic polymer, preferably selected from the group consisting of polyesters, polyamides, polyethers, polyurethanes, polyanhydrides and polyphosphazines, polyesters, polylactides and polycarbonates, poly saccharides, poly(ketals), poly(acetals) and polyacrylates.

Suitable examples of polyesters are poly(□-hydroxy acid)s and polylactones, such as poly (lactic acid), poly (glycolic acid), poly(□-propionolactone), poly (hydroxybutyric acid), poly (valeric acid) or poly(valerolactone), polyhexanoic acid, poly (□-caprolactone), and higher homologues such as poly (□-pentadecalactone) The polyester to be used in the matrix or coating is preferably poly (□-caprolactone) or poly (lactic acid).

Suitable examples of poly(amides) are (protected) □-amino acids, such as poly(leucine), poly(valine), poly(glutamic acid), poly benzyl glutamate and proteins such as elastin, collagen and silk. The poly(amide) to be used in the matrix or coating is preferably poly(glutamic acid) and poly benzyl glutamate.

Suitable examples of poly(anhydrides) are sebacic anhydride, p-(carboxy phenoxy) propane and p-(carboxy phenoxy) hexane.

Suitable examples of poly(ortho esters) are diketene acetals such as 3,9-diethylene 2,4,8,10-tetraoxaspiro[5.5] undecane.

Suitable examples of poly(ethers) are ethylene oxide, propylene oxide, and butylene oxide.

Suitable examples of poly(urethanes) are m-xylylene diisocyanate or hexane diisocyanate and propane diol, ethylene glycol, and poly(ethylene glycol).

Suitable examples of poly(phosphazenes) are amino acid modified dichloro poly(phosphazenes).

Suitable examples of polysaccharides are chitosan, alginate, and cellulose.

Suitable examples of polyacrylates, polymethacrylates and polyacrylamides are acrylic acid, ethyl acrylate, methacrylic acid, N-isopropyl acrylamide, and 2-hydroxyethylmethacrylate.

A suitable example of poly(ketals) is 1,4-phenyleneacetone dimethylene ketal.

Suitable examples of poly(acetals) are oxymethylene and triethylene glycol divinyl ether.

Preferably the hydrophilic parts are selected from the group of materials consisting of poly (ethyleneglycol), poly (lactic acid), poly (□-caprolactone), poly (acrylic acid), poly(glutamic acid).

Preferably, the hydrophobic parts are selected from the group of materials consisting of poly (acrylates), poly (styrene), poly (□-caprolactone), poly (leucine), poly (valine), and poly (carbonates)

In accordance with the present invention the herein the inner part of the capsules is preferably at least partly filled with a disintegration agent which upon release acts to decompose the polymer matrix or initialises or accelerates the decomposition of the polymer matrix. In the case that the capsules are embedded in a coating the disintegration agent acts upon release from the capsules to decompose the coating or initialises or accelerates the decomposition of the coating material.

The decomposed matrix or coating will leave the individual's body through the metabolic pathway or secretion through the kidneys.

The capsules may suitably comprise in addition other components such as antibiotics, enzymes or proteins that can be released upon demand.

Preferably, the disintegration agent which is contained within the shell of the capsule is selected from the group consisting of enzymes, acids, bases, metal complexes, electrolytes and stimuli responsive agents.

Suitable enzymes include lipases, proteases and esterases.

Suitable acids include natural acids, organic acids and poly acids.

Suitable bases include inorganic bases, sodium bicarbonate, and sodium carbonate.

Suitable metal complexes and electrolytes include palladuim compounds, zinc compounds, semerium compounds, ruthenium compounds, magnesium compounds, and lithium compounds.

The present invention also relates to a medical device comprising the bioresorbable composition according to the present invention. Such a medical device can be coated implants such as bone plates, rods, pins, screws or spinal cages that become resorbable after an externally emitted activation signal or coated implants that release functional agents upon activation.

Preferably, the medical device according to the present invention is a bioresorbable implant.

Further, the present invention also relates to a method for the in vivo decomposition of a medical device according to the present invention, which method comprises decomposing at least partly the shells of the capsules by application of an external stimulation source which acts upon the capsule or stimulates an agent which causes disruption of the shells. In the method according to the capsules suitably release a disintegration agent which acts to decompose the polymer matrix or initialises or accelerates the decomposition of the polymer matrix.

The disintegration agent will, for example, by means of hydrolysis decompose the polymer matrix.

The present invention further relates to the use of the bioresorbable composition according to the present invention for the manufacture of a medical device.

The medical device can be manufactured by injection moulding of the core material (potentially including the capsules) and, if relevant for the design, subsequent dip coating of the coating layer including the capsules.

In accordance with the present invention, the external stimulation source comprises a source of magnetic field, electro-magnetic waves or ultra-sound. Preferably, the external stimulation source comprises a source of magnetic field, electro-magnetic waves or ultra-sound. Suitably, the electro-magnetic waves range from radio waves at low frequency (10,000 Hz) to X-rays at high frequency (10¹⁹ Hz). To aid the disruption of the capsules stimuli responsive agent can also be included within the capsule or within the shell of the capsule. A suitable stimuli responsive agent can be Elastin, Gold or Paramagnetic particles.

In addition, the present invention relates to a medical device comprising a body of a first polymer matrix around which a coating layer of a second polymer matrix is applied, wherein the first polymer matrix is soluble in body fluids and the second polymer matrix is essentially insoluble in body fluids, whereby at least part of the coating layer is decomposable by application of an external stimulation source.

The capsules to be used in accordance with the present invention can be made my methods known to the skilled person. They can, for example, be prepared by the spontaneous or forced assembly of polymers with a water insoluble (hydrophobic) and water soluble (hydrophilic) segment or by the layer-by-layer coating of oppositely charged polyelectrolyte on a spherical template. The disintegration agent can be introduced during the preparation of the capsules by dispersing these capsules in a solution containing the disintegrating agent.

The size of the capsules can range of from between 100 nm to 20 micrometers. Preferably, the capsules have a size in the range of from 200 nm to 5 micrometers. Actually, the size of the capsules will depend on thickness of the shell. The thickness of the shell of the capsule can suitably be in the range of from 30 to 50 nanometres.

## Claims

1. A bioresorbable composition comprising a mixture of at least one polymer and capsules, which capsules comprise an inner part which is encapsulated by a shell which shell is at least partly decomposable by application of an external stimulation source.

2. The composition according to claim 1, wherein the mixture has the form of a polymer matrix in which the capsules are embedded.

3. The composition according to claim 1 or 2, wherein at the least one polymer is essentially insoluble in body fluids.

4. The composition according to any one of claims 1-3, wherein the at least one polymer is selected from the group consisting of polyesters, polylactides, polyamides, polyethers, polyurethanes, polycarbonates, polyanhydrides and polyphosphazines.

5. The composition according to claim 4, wherein the at least one polymer is selected from the group consisting of polyesters, polylactides and polycarbonates

6. The composition according to any one of claims 1-5, wherein the shells of the capsules are prepared from a material selected from the group consisting of polyesters, polylactides, polyamides, polyethers, polyurethanes, polycarbonates, polyanhydrides, polyphosphazines, polyacrylates, polymethacrylates, polystyrenics, or a combination thereof.

7. The composition according to claim 6, wherein the material comprises a combination of one or more polyesters, one or more polylactides and one or more polymethacrylates.

8. The composition according to any one of claims 1-7, wherein the inner part of the capsules is at least partly filled with a disintegration agent, which upon release acts, initialises or accelerares decomposition of the polymer matrix.

9. The composition according to claim 8, wherein the disintegration agent is selected from the group consisting of enzymes, acids, bases, metal complexes and electrolytes.

10. A medical device comprising the composition according to any one of claims 1-9.

11. A method for the in vivo decomposition of the medical device according to claim 10 comprising decomposing at least partly the shells of the capsules by application of an external stimulation source.

12. A method according to claim 11, wherein the capsules release a disintegration agent which acts to decompose the polymer matrix.

13. Use of the composition according to any one of claims 1-9 for the manufacture of a medical device.

14. The composition according to any one of claims 1-9, wherein the external stimulation source comprises a source of magnetic waves, electro-magnetic waves or ultra-sound.

15. The method according to claim 11 or 12, wherein the external stimulation source comprises a source of magnetic waves, electro-magnetic waves or ultra-sound.

16. A medical device comprising a body of a first polymer matrix around which a coating layer of a second polymer matrix is applied, wherein the first polymer matrix is soluble in body fluids and the second polymer matrix is essentially insoluble in body fluids, whereby at least part of the coating layer is decomposable by application of an external stimulation source.
